# EUROPEAN PATENT APPLICATION

(11) **EP 2 072 067 A1**
(43) Date of publication of application: **24.06.2009**
(21) Application number: 07025022.0
(22) Date of filing: 21.12.2007
(51) Int. Cl.: A61L 29/12, A61F 2/84

(54) **Lamellar shaped layers in medical devices**

(71) Applicant: Abbott Laboratories Vascular Enterprises Limited, Terrace, Dublin 2 (IE)
(72) Inventor: Lorenz, Günter., 72072 Tübingen (DE); Abendschein, Markus., 72458 Albstadt (DE); Hartwig, Judith., 72415 Grosselfingen (DE); Pschibl, Silke., 72414 Rangendingen (DE); Weidner, Anneliese., 72145 Hirrlingen (DE)
(74) Representative: Peters, Hajo

(57) **Abstract**

The present invention refers to medical devices. Particularly it relates to stent devices and balloon catheter devices. In the most particular aspect of the invention it relates to structures with at least two different lamellar sections used in such a medical device, especially in a balloon on a balloon catheter device carrying a stent comprising at least one layer with at least two lamellar sections different by their shore hardness and its use in a variety of medical procedures to treat medical conditions in animal and human patients.

## Description

### Field of the invention

The present invention refers to medical devices. Particularly it relates to stent devices and balloon catheter devices. In the most particular aspect of the invention it relates to structures with at least two different lamellar sections used in such a medical device, especially in a balloon on a balloon catheter device carrying a stent comprising at least one layer with at least two lamellar sections different by their elasticity, like shore hardness, and its use in a variety of medical procedures to treat medical conditions in animal and human patients.

### Background of the invention

This invention relates to medical devices, especially to stent carrying balloon catheters, for use in angioplasty and other procedures of vessel repair. Angioplasty is an efficient and successful method of opening stenosis in the vascular system.

In a popular form of angioplasty, a balloon catheter is advanced through the vascular system until the balloon, which is carried at the distal end of a catheter shaft, and which may carry an expandable stent, is positioned across the stenosis or damaged vessel. By inflating the balloon pressure is applied to the obstruction which is moved by pressing it against the inner wall of the vessel, whereby the vessel is opened for improved flow. Due to the expansion of the balloon, the stent, which - if used - is situated on the balloon, is also expanded for aiding in repairing the vessel wall and hindering obstruction. The stent is then released by deflating the balloon reducing its circumference until refolding of the balloon. This refolding is a critical step in this form of angioplasty. In some cases the refolding is insufficient leading to deformations, in general called "pan-caking", in which the refolded balloon does not reach the optimal minimum - mostly circular - size, but shows edges and bulges. When withdrawing the balloon catheter with the refolded balloon these bulges and edges might damage the fine vessel of the vascular system.

There are various types of balloon catheters. One type is fed over a guide wire (i.e., "over-the-wire" catheters) and another type serves as its own guide wire ("fixed - wire" catheters). There have been development of variations of these two basic types: the so called "rapid exchange "type, "innerless" catheters, and others. The term "balloon catheter" as defined in this invention is meant to include all the various types of angioplasty catheters which carry a balloon for performing angioplasty and any other type of stent carrying balloon catheter. Balloon catheters also have a wide variety of inner structure, such as different lumen design, of which there are at least three basic types: triple lumen, dual lumen and co-axial lumen. All these varieties of internal structure and design variations are included in the definition "balloon catheter" herein.

If a balloon catheter is used in percutaneous transluminal coronary angioplasty (PTCA), it is typically advanced through a guide catheter to a preselected vessel location, such as the aorta, for example. Using fluoroscopy, the surgeon advances the catheter until the balloon is located across the stenosis or obstruction. This may involve the use of a guide wire over which the catheter is moved or alternatively the catheter may act as its own guide wire.

The use of stents, balloons, catheters, especially balloon catheters and other medical devices etc. in minimal invasive surgery, especially in the cardiovascular field, has - over the last years - shown a high growth. As a consequence the need for modifications to the materials used fulfilling the highly specialized needs in the field of different medicinal devices has clearly risen. Especially in the field of cardiovascularily used balloons there was a clear desire for a modified material showing a suitable compliance, a high burst pressure, but also a good and dependable refolding behaviour, especially avoiding "pan-caking", but also "dog-boning" (an inflation of the balloon outside the stenotic area of the vessel).

The present invention is aimed at guiding the refolding into an optimized form being equal or very similar to the structure of the balloon on the balloon catheter before expanding. The object of the present invention is to improve the refolding behavior of the balloon by providing different lamellar sections in the balloon material having different elasticities, like compliance/shore hardness. This invention thus avoids sub-optimal refolding of the balloon after expansion, especially "pan-caking", associated with prior solutions, but also the dreaded "dog-boning" during balloon expansion.

### Summary of the invention

The present invention is directed in one embodiment to a medical device comprising an expandable and contractible member, which is desirably a medical balloon, with at least one first polymeric-layer consisting of at least one lamellar section A and an equal number of lamellar sections B. These lamellar sections A and B are disposed in an alternating sequence in parallel to the longitudinal axis of the expandable and contractible member, with adjacent sections having a different elasticity, expressed as different shore hardness, E-module, tensile strength, elongation at break, or compliance, but preferably expressed as a different shore hardness. By choosing different elasticity, like shore hardness for the lamellar sections A and B refolding of the expandable and contractible member, which is desirably a medical balloon, is greatly helped. After placing a device, e.g. the stent, in the desired position, e.g. in the coronary vessel in PTCA, using pressure-expansion of the balloon (thus also expanding the stent into the desired state and position), the balloon is deflated. The fact that the sections A and B react differently to the internal pressure of the balloon is also reflected in their behaviour when the balloon is refolding, with the sections with lower elasticity (higher shore hardness) guiding - quasi as a scaffold - the desired refolding ideally into the original form.

In another embodiment the invention is further directed to a medical device being a delivery apparatus (preferably a balloon catheter) comprising a first expandable and contractible member (which desirably is a medical balloon) for delivering at least one second medical device. The second medical device is an expandable medical device disposed about the first expandable and contractible member and desirably is a stent. In this medical device the expandable and contractible member is desirably a medical balloon and has at least one first polymeric-layer consisting of at least one lamellar section A and an equal number of lamellar sections B. These lamellar sections A and B are disposed in an alternating sequence in parallel to the longitudinal axis of the expandable and contractible member, with adjacent sections having a different elasticity, expressed as different shore hardness, E-module, tensile strength, elongation at break, or compliance, but preferably expressed as a different shore hardness.

In yet another embodiment the invention is directed to a method for improving the refolding behaviour of an expandable and contractible member, which desirably is a medical balloon. In this method at least one first polymeric-layer of an expandable and contractible member consisting of at least one lamellar section A and an equal number of lamellar sections B disposed in an alternating sequence in parallel to the longitudinal axis of the expandable and contractible member is provided. The adjacent sections A and B have a different elasticity.

In a further embodiment the invention is directed to a method of producing a medical device according to the invention. In this method the lamellar section/s A and the lamellar section/s B are co-extruded, when producing the first polymeric-layer of the expandable and contractible member.

In another further embodiment the invention is directed to a method of producing a certain embodiment of the medical device according to the invention. In this method the lamellar section/s A and the lamellar section/s B of the expandable and contractible member are either simultaneously or consecutively extruded onto a second polymeric-layer.

In a further embodiment the invention is directed to a method of treatment of a disease, like a cardiovascular disease, especially a stenosis, using in a patient, being a mammal, especially a human, in need thereof a medical device according to the invention, desirably in minimal invasive surgery like PTCA.

In yet another embodiment the invention is directed to the use of a medical device according to the invention for the treatment of a disease, like a cardiovascular disease, especially a stenosis, especially through minimal invasive surgery like PTCA.

### Brief Description of the Drawings:

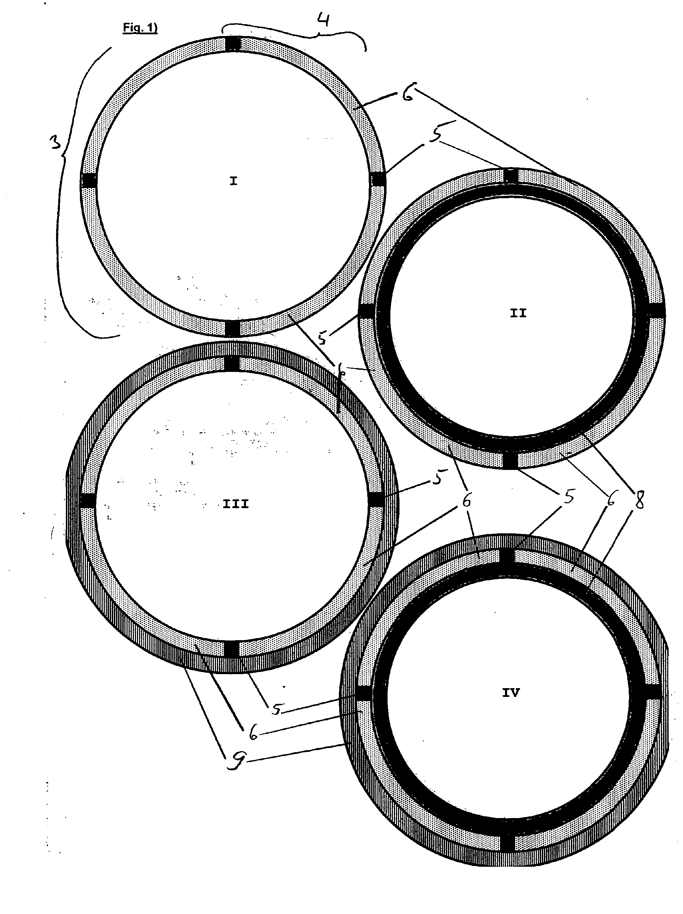

- **Figure 1**: depicts four different lateral cuts cutting at a right angle through the longitudinal axis of the expandable and contractible member (3) showing one embodiment of a first polymeric layer (4) of an expandable and contractible member (3) of a medical device (1) according to the invention. In all cases (I to IV) a layer with 8 lamellar sections is depicted, 4 of them being lamellar sections A (5) and 4 of them being lamellar sections B (6). The different lateral cuts (I to IV) are showing: • (I): the first polymeric layer (4) as a single-layer; • (II): the first polymeric layer (4) with an additional second polymeric-layer (8) disposed within the first polymeric layer (4); • (III): the first polymeric layer (4) with an additional third polymeric-layer (9) disposed about the first polymeric layer (4); • (IV): the first polymeric layer (4) with an additional third polymeric-layer (9) disposed about the first polymeric layer (4) and an additional second polymeric-layer (8) disposed within the first polymeric layer (4).

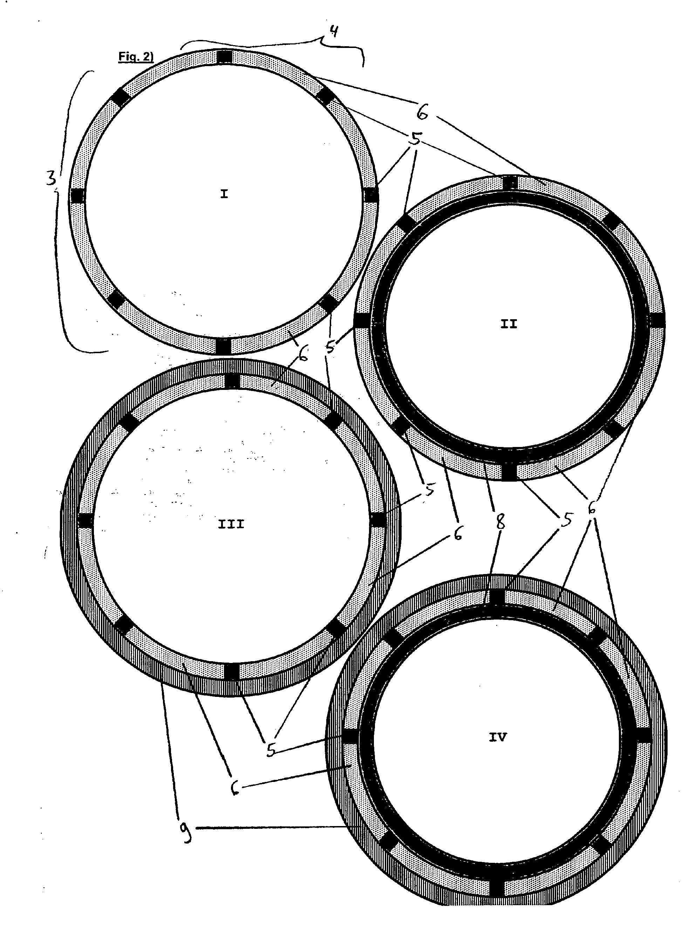

- **Figure 2**: depicts four different lateral cuts cutting at a right angle through the longitudinal axis of the expandable and contractible member (3) showing another embodiment of a first polymeric layer (4) of an expandable and contractible member (3) of a medical device (1) according to the invention. In all cases (I to IV) a layer with 16 lamellar sections is depicted, 8 of them being lamellar sections A (5) and 8 of them being lamellar sections B (6). The different lateral cuts (I to IV) are showing: • (I): the first polymeric layer (4) as a single-layer; • (II): the first polymeric layer (4) with an additional second polymeric-layer (8) disposed within the first polymeric layer (4); • (III): the first polymeric layer (4) with an additional third polymeric-layer (9) disposed about the first polymeric layer (4); • (IV): the first polymeric layer (4) with an additional third polymeric-layer (9) disposed about the first polymeric layer (4) and an additional second polymeric-layer (8) disposed within the first polymeric layer (4).

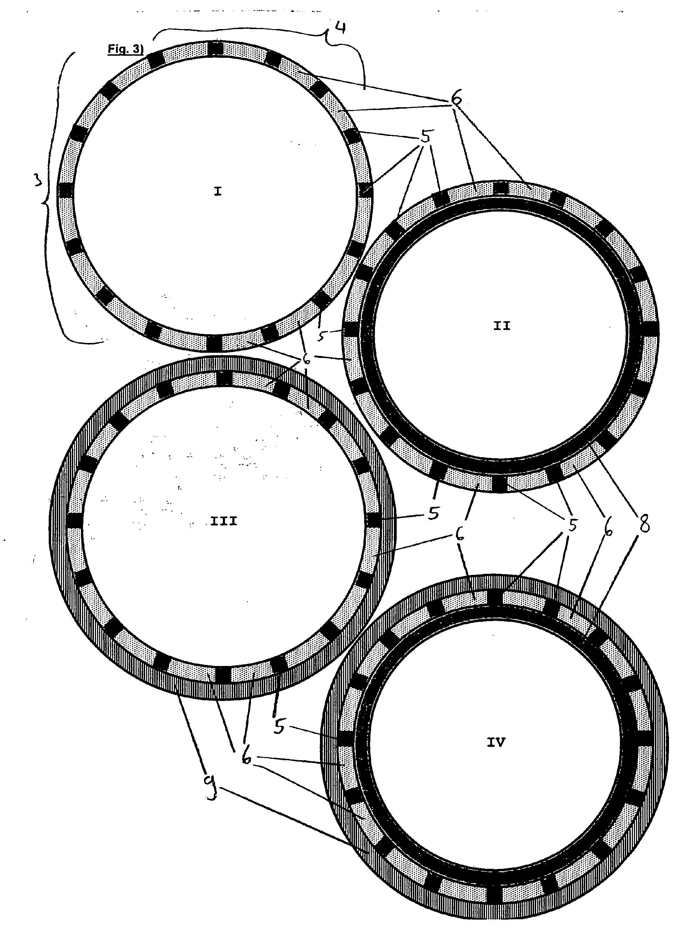

- **Figure 3**: depicts four different lateral cuts cutting at a right angle through the longitudinal axis of the expandable and contractible member (3) showing another embodiment of a first polymeric layer (4) of an expandable and contractible member (3) of a medical device (1) according to the invention. In all cases (I to IV) a layer with 32 lamellar sections is depicted, 16 of them being lamellar sections A (5) and 16 of them being lamellar sections B (6). The different lateral cuts (I to IV) are showing: • (I): the first polymeric layer (4) as a single-layer; • (II): the first polymeric layer (4) with an additional second polymeric-layer (8) disposed within the first polymeric layer (4); • (III): the first polymeric layer (4) with an additional third polymeric-layer (9) disposed about the first polymeric layer (4); • (IV): the first polymeric layer (4) with an additional third polymeric-layer (9) disposed about the first polymeric layer (4) and an additional second polymeric-layer (8) disposed within the first polymeric layer (4).

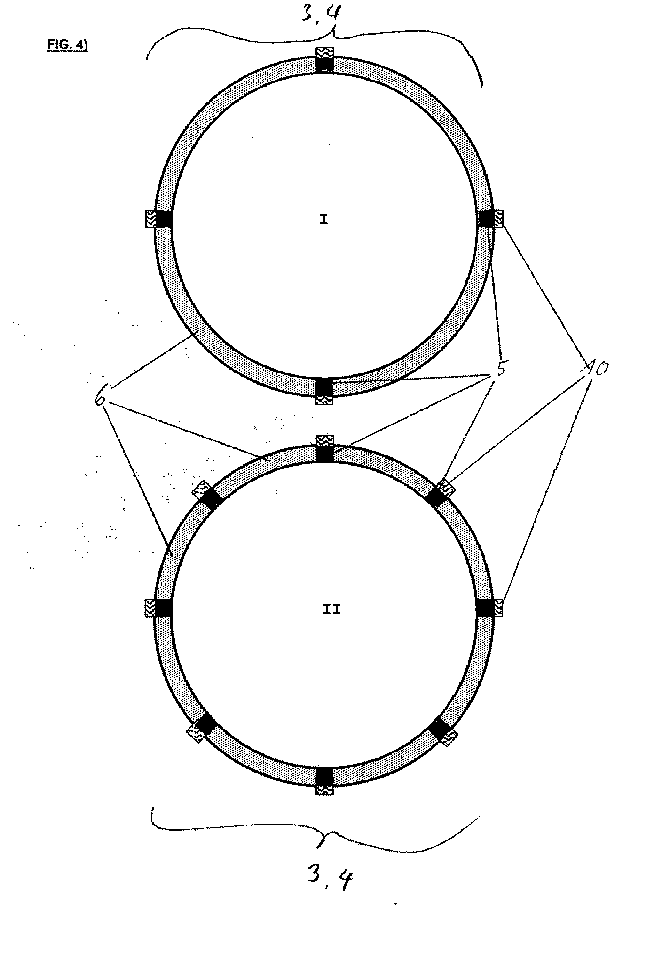

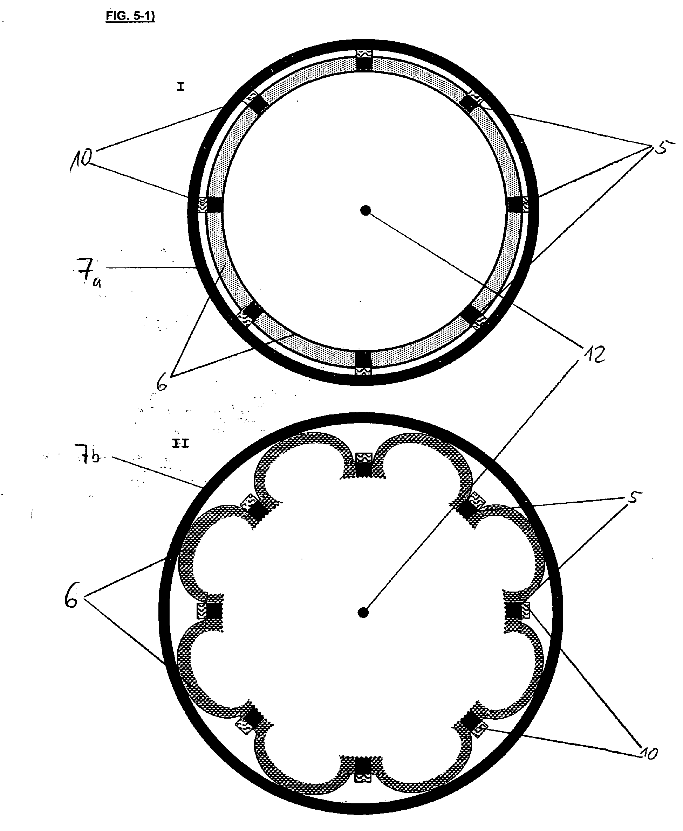

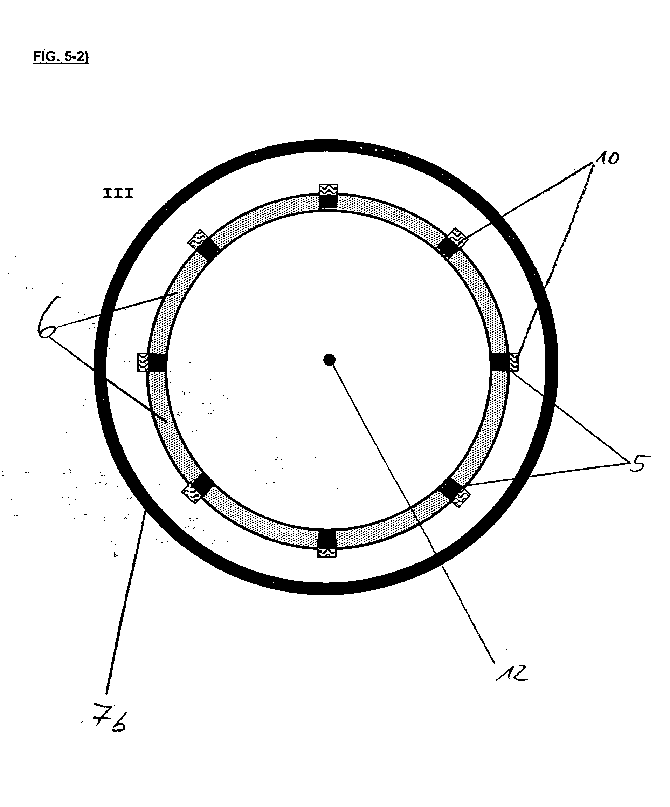

- **Figure 4**: depicts two different lateral cuts cutting at a right angle through the longitudinal axis of the expandable and contractible member (3) showing another embodiment of a first polymeric layer (4) of an expandable and contractible member (3) of a medical device (1) according to the invention. Depicted in (I) is a layer with 8 lamellar sections 4 of them being lamellar sections A (5) and 4 of them being lamellar sections B (6). Depicted in (II) is a layer with 16 lamellar sections 8 of them being lamellar sections A (5) and 8 of them being lamellar sections B (6). In both cases an adhesive area (10) is shown either being an adhesive (10) being added on top of the lamellar section A (5) or symbolising the fact that the polymeric material, of which lamellar section A (5) consists, is adhesive. **Figure 5** is divided into 2 different figures (FIG. 5-1 and FIG. 5-2). It is depicting an embodiment of a medical device (1) according to the invention related to the embodiment depicted in FIG. 4, again showing lateral cuts cutting at a right angle through the longitudinal axis of the expandable and contractible member (3) (preferably a medical balloon) and through a first polymeric layer (4) of an expandable and contractible member (3). In addition the Figure shows an expandable medical device (7) (preferably a stent) in unexpanded (7a) or expanded (7b) state, as well as a core (12), symbolizing the inner core of the medical device (1) (preferably a balloon catheter) and as well the longitudinal axis of the expandable and contractible member (3). An adhesive area (10) is shown either being an adhesive (10) being added on top of the lamellar section A (5) or symbolising the fact that the polymeric material, of which lamellar section A (5) consists, is adhesive. Lamellar section A (5) is (highly) non-compliant, while Lamellar section B (6) is (highly) compliant. Part (I) shows - in abstract form - the situation during introduction of the medical device (1) (a balloon catheter) into a body lumen like a blood vessel. Part (II) shows the stent (7) being expanded by inflating the balloon (3). Part (III) shows the balloon (3) being contracted, while the stent (7) remains in place in expanded state (7b).

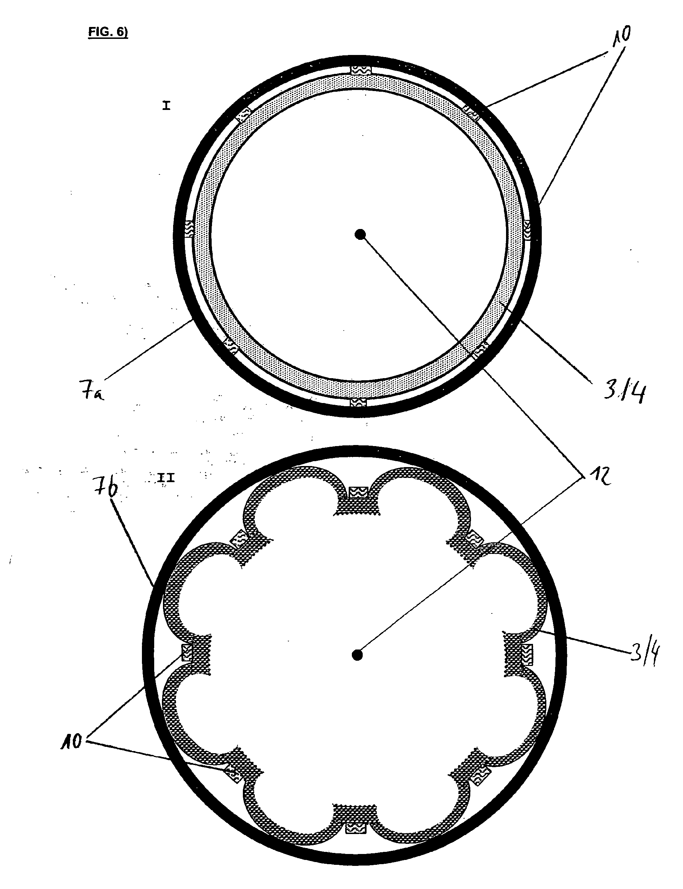

- **Figure 6**: is depicting a different aspect of the invention showing lateral cuts cutting at a right angle through the longitudinal axis of the expandable and contractible member (3) (preferably a medical balloon) and through a first polymeric layer (4) of the expandable and contractible member (3). In addition the Figure shows on the outer surface of the balloon rigid (non-compliant) adhesive stripes (10) between the balloon and the expandable medical device (7) (preferably a stent) in unexpanded (7a) or expanded (7b) state, as well as a core (12), symbolizing the inner core of the medical device (preferably a balloon catheter) and as well the longitudinal axis of the expandable and contractible member (3). Part (I) shows - in abstract form - the situation during introduction of the medical device (1) (a balloon catheter) into a body lumen like a blood vessel. Part (II) shows the stent (7) being expanded by inflating the balloon (3).

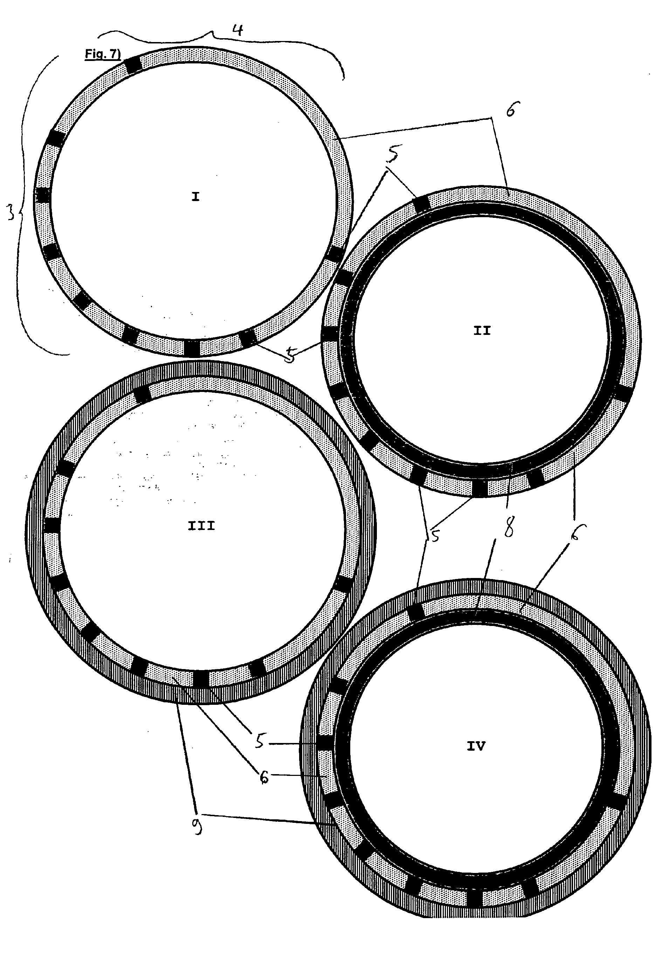

- **Figure 7**: depicts four different lateral cuts cutting at a right angle through the longitudinal axis of the expandable and contractible member (3) showing another embodiment of a first polymeric layer (4) of an expandable and contractible member (3) of a medical device (1) according to the invention. In all cases (I to IV) a layer with 18 lamellar sections is depicted, 9 of them being lamellar sections A (5) and 9 of them being lamellar sections B (6). The distribution of the lamellar sections A (5) and B (6) is non-symmetric allowing for a non-symmetric expansion of the expandable and contractible member (3). The different lateral cuts (I to IV) are showing: • (I): the first polymeric layer (4) as a single-layer; • (II): the first polymeric layer (4) with an additional second polymeric-layer (8) disposed within the first polymeric layer (4); • (III): the first polymeric layer (4) with an additional third polymeric-layer (9) disposed about the first polymeric layer (4); • (IV): the first polymeric layer (4) with an additional third polymeric-layer (9) disposed about the first polymeric layer (4) and an additional second polymeric-layer (8) disposed within the first polymeric layer (4).

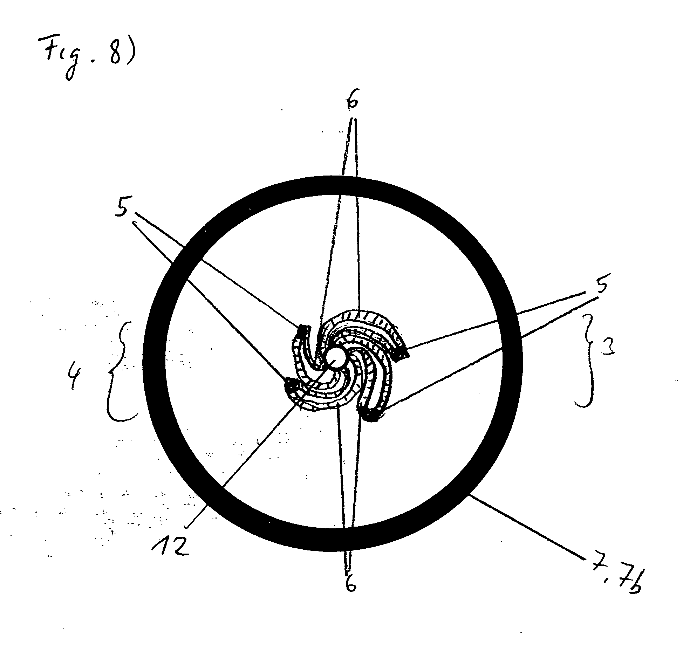

- **Figure 8**: is depicting a different aspect of the invention showing lateral cuts cutting at a right angle through the longitudinal axis of the expandable and contractible member (3) (preferably a medical balloon) and through a first polymeric layer (4) of the expandable and contractible member (3). Deviating from the other figures this figure shows the expandable and contractible member (3) (the medical balloon) in refolded form and the expandable medical device (7) (preferably a stent) in expanded (7b) state, as well as a core (12), symbolizing the inner core of the medical device (preferably a balloon catheter) and as well the longitudinal axis of the expandable and contractible member (3). This figure shows the complete refolding of the expandable and contractible member (3) (the medical balloon) having been successful due to the help of the 4 lamellar sections A (5) having a lower elasticity, a higher shore hardness, than lamellar sections B (6) and thus acting as a scaffold have helped refolding. The medical device (1) (the balloon catheter) with the refolded balloon (3) is about to be removed from the lumen of the expanded stent (7b).

### Detailed Description of the Invention

While the invention may be embodied in many different forms, there are described in detail herein specific preferred embodiments of the invention. This description is an exemplification of the principles of the invention and is not intended to limit the invention to the particular embodiments illustrated.

In one embodiment as depicted in Figs. 1-4 the instant invention is directed to a medical device (1) comprising an expandable and contractible member (3), wherein the expandable and contractible member (3) is comprising at least one first polymeric-layer (4) consisting of at least one lamellar section A (5) and an equal number of lamellar sections B (6) disposed in an alternating sequence in parallel to the longitudinal axis of the expandable and contractible member (3) with adjacent sections having a different elasticity. This elasticity desirably is expressed as different shore hardness, E-module, tensile strength, elongation at break, or compliance, but preferably is expressed as a different shore hardness. This invention allows by having the different elasticity, like shore hardness an improved refolding behaviour of the expandable and contractible member (3), thus - avoiding "pan-caking" upon deflation, but also avoiding "dog-boning" during inflation - allowing save removal/movement of the medical device through a tight lumen, like a vessel

As a general remark elasticity (of the polymer) as used herein can be expressed in different ways like shore hardness, E-module, tensile strength, elongation at break, or compliance, but in the context of this invention preferably is expressed as different shore hardness, even though the other expressions for elasticity like E-module, tensile strength, elongation at break, or compliance may also be used.

In this embodiment the expandable and contractible member (B) desirably is a medical balloon. The medical balloon (B) is capable of being expanded and contracted. Desirably the first polymeric-layer (4) of the first expandable and contractible member (3), the medical balloon, is consisting of at least 2 each of lamellar sections A (5) and lamellar sections B (6). In another embodiment desirably the first polymeric-layer (4) of the first expandable and contractible member (3), the medical balloon, is consisting of at least 3 each of lamellar sections A (5) and lamellar sections B (6). Desirably lamellar section A (5) and lamellar section B (6) are consisting of two different polymeric materials, especially different polymers or block-co-polymers like selected from e.g. Nylons, PEBA or mixtures thereof.

In one embodiment of the medical device according to the invention desirably the expandable and contractible member (3), the medical balloon, is comprising an additional second polymeric-layer (8) disposed within the first polymeric layer (4). In another alternative embodiment the expandable and contractible member (3) is comprising an additional third polymeric-layer (9) disposed about the first polymeric layer (4), and in a third alternative embodiment the expandable and contractible member (3) is comprising an additional third polymeric-layer (9) disposed about the first polymeric layer (4) and an additional second polymeric-layer (8) disposed within the first polymeric layer (4). Desirably the second polymeric-layer (8) in another alternative embodiment the second (8) and/or the third polymeric layer (9) is consisting of PEBA or Nylon or mixtures thereof. Advantageously the second (or third) polymeric layer in this embodiment provides the strength and air tightness to the medical balloon, allowing the sections A and B to not necessarily having to be tightly bound to each other and also to be selected from material usually not used in a medical balloon.

In another embodiment of the medical device according to the invention as depicted for example in Fig. 5 desirably the polymeric material, of which lamellar section A (5) or lamellar section B (6) consists, is adhesive, while the other is not, or one of lamellar section A (5) or lamellar section B (6) is layered with an adhesive (10), while the other is not. Adhesives as defined in this invention especially are mild adhesives, preferably an adhesive being pressure sensitive and selected as to release a second medical device (stent) (7) disposed on the expandable and contractible member (3) (the medical balloon) from the expandable and contractible member (3) upon pressure being applied from within the second medical device aiming at distancing the stent (7) from the adhesive (10) of the balloon. Selected pressure sensitive adhesives, include silicone type pressure sensitive adhesives, acrylic type pressure sensitive adhesives and urethane type pressure sensitive adhesives. Examples of acrylic type pressure sensitive adhesives include NeoTac A-580, NeoTac A-574, NeoTac 2010, NeoTac 2457, NeoTac 2465, NeoTac 5468 all from Zeneca Resins. An example of an urethane type pressure sensitive adhesive is NeoTac 560 (Zeneca Resins). Desirably the pressure sensitive adhesive will have good water resistance to ensure good adhesion when the stent and the balloon are in contact with body fluids. In addition desirably the polymeric material, of which one of lamellar sections A (5) or B (6), especially A (5) consists, is non-compliant while the other polymeric material, of which the other of lamellar sections A (5) or B (6) consists, is compliant. Possible materials could involve (hard) Nylon (polyamides) and for the more soft/compliant part certain sorts of PEBA.

As a general remark "adhesive" as defined in this application is encompassing all forms of interaction between surfaces transferring adhesion between them includingfriction by a rough surface or simply "tacky" surfaces provided by certain sorts of polymeric material like e.g. certain sorts of PEBA.

As a general information and with the focus of this invention on balloon material for balloon catheters one of the main parameters of a balloon is compliance, the change of the balloon diameter with rising inflation pressure; as used herein three categories are being identified:
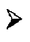 Non-compliant (NC) with a diameter increase of up to 0.55% per bar;
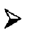 Semi-compliant (SC) with a diameter increase of between 0.55 to 0.8 % per bar;
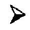 Compliant with a diameter increase over 0.8 % per bar
as the balloon is pressurized from an inflation pressure between the nominal pressure and rated burst pressure.

While a certain level of compliance is needed to allow the compression of the arterio-sclerotic plaque in a vessel, an amount of pressure expressed on the stenosis as executed by a more non-compliant balloon is also needed. Also semi-compliant and compliant balloons are more prone to failure during PTCA and also "dog-boning", an inflation of the balloon outside the stenotic area of the vessel resulting sometimes in devastating stress on the healthy part of the vessel.

Another main parameter of a balloon in a balloon catheter device is burst pressure, the pressure a balloon in a balloon catheter device can withstand from within before bursting. While a certain degree of pressure expressed on the stenosis is a clear necessity for the function of a balloon catheter device the risks set to this pressure by the obviously devastating results of a possible burst of the balloon while in a lumen, e.g. of a vessel, do considerably limit the options given to a practitioner in using this device. Thus, also a high resistance to burst pressure is mostly a wanted effect in the balloon of a balloon catheter device.

In one embodiment the medical device (D) is a delivery apparatus for delivering at least one second medical device. Desirably the medical device is a delivery apparatus with a stent, a stent graft, a graft or a graft connector as second medical device. Very desirably the medical device is a delivery apparatus with a stent as second medical device. In one embodiment this delivery apparatus comprises a catheter with a medical balloon (3) as expandable and contractible member (3), thus being a balloon catheter. Thereby the second medical device, the stent (7), is disposed about the medical balloon (3) (the first expandable and contractible member). Additional details concerning the construction of suitable stent delivery apparatuses for use in the invention may be found in U.S. Pat. Nos. 6,036,697, 5,893,868 and 5,957,930 and elsewhere in the patent literature. Any suitable stent may be used whether formed of metal or of polymeric material or of another material. Examples of suitable stents may be found in US 6,533,809 and US 6,602,285.

The Medical balloon (3) is capable of being expanded and contracted. Desirably the first polymeric-layer (4) of the first expandable and contractible member (3), the medical balloon, is consisting of at least 2 each of lamellar sections A (5) and lamellar sections B (6). In another embodiment desirably the first polymeric-layer (4) of the first expandable and contractible member (3), the medical balloon, is consisting of at least 3 each of lamellar sections A (5) and lamellar sections B (6).

Desirably lamellar section A (5) and lamellar section B (6) are consisting of two different polymeric materials, especially different polymers or block-co-polymers like selected from e.g. Nylons, PEBA or mixtures thereof.

"Balloon", "Medical Balloon" or "balloon material" in the context of this invention especially means a balloon like those used in balloon angioplasty and the material used for these balloons, especially balloon catheters. In this, e.g. a balloon catheter is inserted into an artery or other lumen and advanced to e.g. a narrowing in a coronary artery. The balloon is then inflated by gas or fluids to enlarge the lumen and/or - often - to place a medical device.

"Stent" means an elongate implant with a hollow interior and at least two orifices and usually a circular or elliptical, but also any other, cross section, preferably with a perforated, lattice-like structure that is implanted into vessels, in particular blood vessels, to restore and maintain the vessels patent and functional.

"Graft" means an elongate implant with a hollow interior and with at least two orifices and usually circular or elliptical, but also any other, a cross section and with at least one closed polymer surface which is homogeneous or, optionally, woven from various strands. The surface preferably is impermeable to corpuscular constituents of blood and/or for water, so that the implant serves as a vascular prosthesis and is usually employed for damaged vessels or in place of vessels.

"Stent graft" means a connection between a stent and a graft. A stent graft preferably comprises a vascular prosthesis reinforced with a stent (both as defined above), wherein a polymer layer is homogeneous or, optionally, woven, knitted plaited etc. from various strands and is either impermeable for corpuscular constituents of blood and/or for water or can also be permeable. More preferably, the stent has on at least 20% of its surface a perforated (lattice-like), preferably metallic, outer layer and at least one closed polymer layer that is located inside or outside the stent outer layer. The closed polymer layer may be homogeneous or, optionally, woven from various strands, and is impermeable for corpuscular constituents of blood and/or for water. Optionally, where the closed polymer layer is disposed inside the metallic outer layer, a further perforated (lattice-like), preferably metallic, inner layer may be located inside the polymer layer.

"Graft connector" means an implant that connects at least two hollow organs, vessels or grafts, consists of the materials defined for grafts or stent grafts and/or has the structure defined for the latter. Preferably, a graft connector has at least two, three or four, orifices, arranged, for example, as an asymmetric "T" shape.

"Catheter" means a tubular instrument intended for introduction into hollow organs. More preferably, a catheter may be designed for use in guiding other catheters, or for angiography, ultrasound imaging, or - especially - balloon catheters for dilatation or stent delivery. This includes also a "Catheter pump" meaning a catheter provided on its tip with a propeller able to assist the pumping of the myocardium.

In one embodiment of the medical device as a delivery apparatus for delivering at least one second medical device, especially a stent, according to the invention desirably the expandable and contractible member (3), the medical balloon, is comprising an additional second polymeric-layer (8) disposed within the first polymeric layer (4). In another alternative embodiment the expandable and contractible member (3) is comprising an additional third polymeric-layer (9) disposed about the first polymeric layer (4), and in a third alternative embodiment the expandable and contractible member (3) is comprising an additional third polymeric-layer (9) disposed about the first polymeric layer (4) and an additional second polymeric-layer (8) disposed within the first polymeric layer (4). Desirably the second polymeric-layer (8) in another alternative embodiment the second (8) and/or the third polymeric layer (9) is consisting of PEBA or Nylon or mixtures thereof. Advantageously the second (or third) polymeric layer in this embodiment provides the strength and air tightness to the medical balloon, allowing the sections A and B to not necessarily having to be tightly bound to each other and also to be selected from material usually not used in a medical balloon.

In this embodiment of the medical device as a delivery apparatus for delivering at least one second medical device, especially a stent, according to the invention desirably the polymeric material, of which lamellar section A (5) or lamellar section B (6) consists, is adhesive, while the other is not, or one of lamellar section A (5) or lamellar section B (6) is layered with an adhesive (10), while the other is not. Adhesives as defined in this invention especially are mild adhesives, preferably an adhesive being pressure sensitive and selected as to release the second medical device (stent) (7) from the expandable and contractible member (3) (the medical balloon) upon pressure being applied from within the second medical device aiming at distancing the stent (7) from the adhesive (10) of the balloon. Selected pressure sensitive adhesives, include silicone type pressure sensitive adhesives, acrylic type pressure sensitive adhesives and urethane type pressure sensitive adhesives. Examples of acrylic type pressure sensitive adhesives include NeoTac A-580, NeoTac A-574, NeoTac 2010, NeoTac 2457, NeoTac 2465, NeoTac 5468 all from Zeneca Resins. An example of an urethane type pressure sensitive adhesive is NeoTac 560 (Zeneca Resins). Desirably the pressure sensitive adhesive will have good water resistance to ensure good adhesion when the stent and the balloon are in contact with body fluids. In addition desirably the polymeric material, of which one of lamellar sections A (5) or B (6), especially A (5) consists, is non-compliant while the other polymeric material, of which the other of lamellar sections A (5) or B (6) consists, is compliant. Possible materials could involve (hard) Nylon (polyamides) and for the more soft/compliant part certain sorts of PEBA.

Another aspect and embodiment of the current invention is directed to a method for improving the refolding behaviour of a first expandable and contractible member (3) of a medical device (1) comprising providing at least one first polymeric-layer (4) consisting of at least one lamellar section A (5) and an equal number of lamellar sections B (6) disposed in an alternating sequence in parallel to the longitudinal axis of the expandable and contractible member (3) with adjacent sections having a different elasticity. Desirably the elasticity may be expressed as different shore hardness, E-module, tensile strength, elongation at break, or compliance, but preferably is expressed as a different shore hardness.

In this embodiment of a method for improving the refolding behaviour of a first expandable and contractible member (3) of a medical device (1) desirably the first expandable and contractible member (3) is a medical balloon. The Medical balloon (3) is capable of being expanded and contracted. Desirably the first polymeric-layer (4) of the first expandable and contractible member (3), the medical balloon, is consisting of at least 2 each of lamellar sections A (5) and lamellar sections B (6). Also desirably the first polymeric-layer (4) of the first expandable and contractible member (3), the medical balloon, is consisting of at least 3 each of lamellar sections A (5) and lamellar sections B (6). Desirably lamellar section A (5) and lamellar section B (6) are consisting of two different polymeric materials, especially different polymers or block-co-polymers like selected from e.g. Nylons, PEBA or mixtures thereof.

In one embodiment of a method for improving the refolding behaviour of a first expandable and contractible member (3) of a medical device (1), desirably there is provided an additional second polymeric-layer (8) of the expandable and contractible member (3) disposed within the first polymeric layer (4). In another alternative embodiment desirably there is provided an additional third polymeric-layer (9) disposed about the first polymeric layer (4), and in a third alternative embodiment desirably there is provided an additional third polymeric-layer (9) of the expandable and contractible member (3) disposed about the first polymeric layer (4) and an additional second polymeric-layer (8) of the expandable and contractible member (3) disposed within the first polymeric layer (4). Desirably the second polymeric-layer (8), in another alternative embodiment the second (8) and/or the third polymeric layer (9) is consisting of PEBA or Nylon or mixtures thereof. Advantageously the second (or third) polymeric layer in this embodiment provides the strength and air tightness to the medical balloon, allowing the sections A and B to not necessarily having to be tightly bound to each other and also to be selected from material usually not used in a medical balloon.

In one embodiment of a method for improving the refolding behaviour of a first expandable and contractible member (3) of a medical device (1), desirably the polymeric material, of which lamellar section A (5) or lamellar section B (6) consists, is adhesive, while the other is not, or one of lamellar section A (5) or lamellar section B (6) is layered with an adhesive (10), while the other is not. Adhesives as defined in this invention especially are mild adhesives , preferably an adhesive being pressure sensitive and selected as to release the second medical device (stent) (7) from the expandable and contractible member (3) (the medical balloon) upon pressure being applied from within the second medical device aiming at distancing the stent (7) from the adhesive (10) of the balloon. Selected pressure sensitive adhesives, include silicone type pressure sensitive adhesives, acrylic type pressure sensitive adhesives and urethane type pressure sensitive adhesives. Examples of acrylic type pressure sensitive adhesives include NeoTac A-580, NeoTac A-574, NeoTac 2010, NeoTac 2457, NeoTac 2465, NeoTac 5468 all from Zeneca Resins. An example of an urethane type pressure sensitive adhesive is NeoTac 560 (Zeneca Resins). Desirably the pressure sensitive adhesive will have good water resistance to ensure good adhesion when the stent and the balloon are in contact with body fluids. In addition desirably the polymeric material, of which one of lamellar sections A (5) or B (6), especially A (5) consists, is non-compliant while the other polymeric material, of which the other of lamellar sections A (5) or B (6) consists, is compliant. Possible materials could involve (hard) Nylon (polyamides) and for the more soft/compliant section certain sorts of PEBA.

In one further embodiment of a method for improving the refolding behaviour of a first expandable and contractible member (3) of a medical device (1) comprising providing at least one first polymeric-layer (4) consisting of at least one lamellar section A (5) and an equal number of lamellar sections B (6) disposed in an alternating sequence in parallel to the longitudinal axis of the expandable and contractible member (3) with adjacent sections having a different elasticity, like shore hardness, the medical device (1) is a delivery apparatus for delivering at least one second medical device (7). The expandable and contractible member (3) is comprising at least one first polymeric-layer (4) consisting of at least one lamellar section A (5) and an equal number of lamellar sections B (6) disposed in an alternating sequence in parallel to the longitudinal axis of the expandable and contractible member (3) with adjacent sections having a different elasticity, like shore hardness. Desirably the medical device (7) is a delivery apparatus with a stent, a stent graft, a graft or a graft connector as second medical device. Very desirably the medical device (1) is a delivery apparatus with a stent (7) as second medical device. In one embodiment of a method for improving the refolding behaviour of a first expandable and contractible member (3) of a medical device (1) this delivery apparatus comprises a catheter with a medical balloon (3) as first expandable and contractible member, thus being a balloon catheter. Thereby the second medical device, the stent (7), is disposed about the medical balloon (3) (the first expandable and contractible member). Additional details concerning the construction of suitable stent delivery apparatuses for use in the invention may be found in U.S. Pat. Nos. 6,036,697, 5,893,868 and 5,957,930 and elsewhere in the patent literature. Any suitable stent may be used whether formed of metal or of polymeric material or of another material. Examples of suitable stents may be found in US 6,533,809 and US 6,602,285. The Medical balloon is capable of being expanded and contracted. In this embodiment of this method for improving the refolding behaviour of a first expandable and contractible member (3) of a medical device (1) desirably the polymeric material, of which lamellar section A (5) or lamellar section B (6) consists, is adhesive, while the other is not, or one of lamellar section A (5) or lamellar section B (6) is layered with an adhesive (10), while the other is not. Adhesives as defined in this invention especially are mild adhesives, preferably an adhesive being pressure sensitive and selected as to release the second medical device (stent) (7) from the expandable and contractible member (3) (the medical balloon) upon pressure being applied from within the second medical device aiming at distancing the stent (7) from the adhesive (10) of the balloon. Selected pressure sensitive adhesives, include silicone type pressure sensitive adhesives, acrylic type pressure sensitive adhesives and urethane type pressure sensitive adhesives. Examples of acrylic type pressure sensitive adhesives include NeoTac A-580, NeoTac A-574, NeoTac 2010, NeoTac 2457, NeoTac 2465, NeoTac 5468 all from Zeneca Resins. An example of an urethane type pressure sensitive adhesive is NeoTac 560 (Zeneca Resins). Desirably the pressure sensitive adhesive will have good water resistance to ensure good adhesion when the stent and the balloon are in contact with body fluids. In addition desirably the polymeric material, of which one of lamellar sections A (5) or B (6), especially A (5) consists, is non-compliant while the other polymeric material, of which the other of lamellar sections A (5) or B (6) consists, is compliant. Possible materials could involve (hard) Nylon (polyamides) and for the more soft/compliant part certain sorts of PEBA.

Another aspect and embodiment of the current invention is directed to a method of producing a medical device according to the invention, wherein the lamellar section/s A (5) and the lamellar section/s B (6) are co-extruded, when producing the first polymeric-layer (4) of the expandable and contractible member (3).

Another aspect and embodiment of the current invention is directed to a method of producing a medical device according to the invention, wherein the lamellar section/s A (5) and the lamellar section/s B (6) of the expandable and contractible member (3) are either simultaneously or consecutively extruded onto the second polymeric-layer (8).

Another aspect and embodiment of the current invention is directed to a method of treatment of a disease, like a cardiovascular disease, especially a stenosis, using in a patient, being a mammal, especially a human, in need thereof a medical device (1) according to the invention, desirably in minimal invasive surgery like PTCA. In this, the first expandable and contractible member (3) (the Medical Balloon) comprising at least one first polymeric-layer (4) consisting of at least one lamellar section A (5) and an equal number of lamellar sections B (6) disposed in an alternating sequence in parallel to the longitudinal axis of the expandable and contractible member (3) with adjacent sections having a different elasticity, like shore hardness, can be advantageously used, having an improved refolding behaviour allowing save removal/movement of the medical device through a tight lumen, like a vessel.

A further aspect and embodiment of the current invention is directed to the use of a medical device (1) according to the invention for the treatment of a disease, like a cardiovascular disease, especially a stenosis, especially through minimal invasive surgery like PTCA. In this, the first expandable and contractible member (3) (the Medical Balloon) comprising at least one first polymeric-layer (4) consisting of at least one lamellar section A (5) and an equal number of lamellar sections B (6) disposed in an alternating sequence in parallel to the longitudinal axis of the expandable and contractible member (3) with adjacent sections having a different elasticity, like shore hardness, can be advantageously used, allowing save removal/movement of the medical device through a tight lumen, like a vessel.

### Example:

### Example 1: Balloon with one layer and different lamellar sections A and B being co-extruded during balloon extrusion.

In this embodiment of the invention - depicted in general outlines Figs. 1 (I), 2 **(I),** and 3 **(I)** the medical device (1) is a balloon catheter with one (first) polymeric layer (4) and accordingly the expandable and contractible member (3) a medical balloon. During hot-melt extrusion of the balloon (3) the tubing of the balloon (3) is extruded lamellar with two materials of different elasticity - shore hardness - in lamellar sections A (5) and B (6) parallel to the longitudinal axis of the balloon (3). In these concrete examples 3 or 5 lamellar sections A are alternating with 3 or 5 lamellar sections B. The materials used are: for lamellar section A Nylon 12 of a high shore hardness and for lamellar section B the material is a PEBAX^{®} with a lower shore hardness. After extrusion the balloon is blown in a blow molding form. The balloon is then removed, crimped and folded on a catheter. Afterwards a metal stent (7) in non-extended state (7a) is crimped onto the folded balloon (3). If considered necessary the balloon catheter may then be treated with a lubricious material such as silicones or hydrogel polymers as well as PEO (Polyethylene oxide), NPG (neopentyl glycol diacrylate).
After positioning the expanded stent (7b) in the vascular system by inflating the balloon (3) the balloon (3) is being contracted. The lamellar sections A (5) are having a higher shore hardness as the lamellar sections B (6) with a lower shore hardness. Accordingly the sections A help the refolding of the balloon giving a scaffold to the overall structure which on the other hand is more flexible due to the softer lamellar section B, thus avoiding the dreaded "pan-caking" upon deflation, but also avoiding "dog-boning" during inflation.

### Example 2a: Balloon with one layer having different lamellar section A and B and a second layer within the first layer with the first layer being layered consecutively onto the second layer.

In this embodiment of the invention - depicted in general outlines Figs. 1 (II), 2 (II), and 3 (II) - the medical device (1) is a balloon catheter with one (first) polymeric layer (4) and a (second) inner polymeric layer (8). Accordingly the expandable and contractible member (3) is a medical balloon. First an inner layer (8) of the balloon (3) is formed of a standard polymer like Nylon 12 or PEBAX^{®} 7033 having the necessary strength and ability for the medical balloon being used in PTCA. Thenlamellar stripes of material is layered in lamellar sections A (5) and B (6) parallel to the longitudinal axis of the balloon (3) on this inner layer (8). The big advantage is here that the lamellar sections A and B do not need to be bound to each other to give the necessary tightness and strength to the medical balloon (3) this being already conferred by the inner layer (8). After forming the layers, the balloon is blown in a blow molding form. The balloon is then removed, crimped and folded on a catheter. Afterwards a metal stent (7) in non-extended state (7a) is crimped onto the folded balloon (3). If considered necessary the balloon catheter may then be treated with a lubricious material such as silicones or hydrogel polymers as well as PEO (Polyethylene oxide), NPG (neopentyl glycol diacrylate). After positioning the expanded stent (7b) in the vascular system by inflating the balloon (3) the balloon (3) is being contracted. The lamellar sections A (5) are having a higher shore hardness as the lamellar sections B (6) with a lower shore hardness. Accordingly the sections A help the refolding of the balloon giving a scaffold to the overall structure which on the other hand is more flexible due to the softer lamellar section B, thus avoiding the dreaded "pan-caking", but also "dog-boning".

### Example 2b: Balloon with one layer having different lamellar section A and B and a second layer within the first layer with the first layer and the second layer being extruded in parallel.

In this embodiment of the invention following closely to example 2a - depicted in general outlines Figs. 1 (II), 2 (II), and 3 (II) - the medical device (1) is a balloon catheter with one (first) polymeric layer (4) and a (second) inner polymeric layer (8). Accordingly the expandable and contractible member (3) is a medical balloon. In parallel the inner layer (8) of the balloon (3) formed of a standard polymer like Nylon 12 or PEBAX^{®} 7033 having the necessary strength and ability for the medical balloon being used in PTCA is extruded in parallel to the lamellar stripes of lamellar sections A (5) and B (6) of the first polymeric layer (4) being extruded in a parallel pattern to the longitudinal axis of the balloon (3) onto this inner layer (8). The advantage is here that the lamellar sections A and B do not need to be bound to each other to give the necessary tightness and strength to the medical balloon (3) this being already conferred by the inner layer (8). After forming the layers, the balloon is blown in a blow molding form. The balloon is then removed, crimped and folded on a catheter. Afterwards a metal stent (7) in non-extended state (7a) is crimped onto the folded balloon (3). If considered necessary the balloon catheter may then be treated with a lubricious material such as silicones or hydrogel polymers as well as PEO (Polyethylene oxide), NPG (neopentyl glycol diacrylate). After positioning the expanded stent (7b) in the vascular system by inflating the balloon (3) the balloon (3) is being contracted. The lamellar sections A (5) are having a higher shore hardness as the lamellar sections B (6) with a lower shore hardness. Accordingly the sections A help the refolding of the balloon giving a scaffold to the overall structure which on the other hand is more flexible due to the softer lamellar section B, thus avoiding the dreaded "pan-caking", but also "dog-boning".

### Example 3: Balloon with one layer and different lamellar section A and B with a high difference in compliance and with Sections A carrying a a) pressure sensitive adhesive or b) a stripe of soft PEBAX^{®}

In this embodiment of the invention - depicted in Fig 5 (Fig 5-1 and Fig 5-2) - the medical device (1) according to the invention is a balloon catheter for stent delivery with the expandable contractible member (3) accordingly being a medical balloon. The balloon is having one (first) polymeric-layer (4). The polymeric layer is consisting of lamellar sections A (5) and lamellar sections B (6) co-extruded in an alternating sequence in parallel to the longitudinal axis of the balloon during hot-melt extrusion of the polymeric materials. The materials used are: for lamellar section A Nylon 12 of a high shore hardness and accordingly causing non-compliance in their respective area of the balloon, and for lamellar section B the material is a soft PEBAX^{®} with a low shore hardness thus causing a high compliance of the section of the balloon which is formed by section B.
**In version a)** with the pressure sensitive adhesive after blow molding in a blow molding form, the lamellar section A is covered with an adhesive (10) being pressure sensitive and selected as to release the second medical device (stent) (7) from the expandable and contractible member (3) (the medical balloon) upon pressure being applied from within the second medical device aiming at distancing the stent (7) from the adhesive (10) of the balloon. Selected pressure sensitive adhesives, include silicone type pressure sensitive adhesives, acrylic type pressure sensitive adhesives and urethane type pressure sensitive adhesives. Examples of acrylic type pressure sensitive adhesives include NeoTac A-580, NeoTac A-574, NeoTac 2010, NeoTac 2457, NeoTac 2465, NeoTac 5468 all from Zeneca Resins. An example of an urethane type pressure sensitive adhesive is NeoTac 560 (Zeneca Resins). Desirably the pressure sensitive adhesive will have good water resistance to ensure good adhesion when the stent and the balloon are in contact with body fluids. The balloon is then removed and crimped and folded on a catheter, thereby especially exposing the adhesive surface (10) on lamellar section A (5).
**In version b)** with the soft PEBAX^{®} the soft PEBAX^{®} is extrude onto the lamellar section A. Following that the balloon is blown in a blow molding form. The balloon is then removed and crimped and folded on a catheter, thereby especially exposing the adhesive surface (10), the sticky soft PEBAX^{®}, on lamellar section A (5).
**In both versions a) and b)** afterwards a metal stent (7) in non-extended state (7a) is then crimped onto the folded balloon (3), thereby allowing the adhesive surface (10) to adhere to the inner surface of the metal stent (7, 7a). If considered necessary the balloon catheter may then be treated with a lubricious material such as silicones or hydrogel polymers as well as PEO (Polyethylene oxide) , NPG (neopentyl glycol diacrylate).

When introducing the balloon catheter into the vascular system the lubricious coating - if any - is facilitating movement in the system, while the stent remains fixed on the balloon stopping it from slipping-off during advancement (e.g. over a guide wire) due to the adhesive surface (10). So in Fig. 5-1, (I) shows - in abstract form - the situation during introduction of the medical device (1) (the balloon catheter) into a body lumen like a blood vessel. An expandable medical device (7) (a stent) in unexpanded state (7a) is disposed about the medical balloon (3). While being moved, the adhesive (10) on the lamellar section A (5) is fixing the stent (7) to the medical balloon (3), thus avoiding any slipping of the stent (7) from the balloon (3).

As illustrated in Fig. 5-1 (II), when reaching the intended position in the vascular system the stent (7) is expanded (7b) by inflating the balloon (3). Thereby, the lamellar sections A and B of the first polymeric layer (4) do behave differently transferring a different compliance to the sections of the balloon they are forming. The lamellar sections B (6) are expanding considerably, being highly compliant and thus are expanding more and beyond the lamellar section A (5) which is highly non-compliant. By this expansion of the compliant lamellar sections B (6) beyond the radius reached by the non-compliant lamellar sections A (5) the adhesive connection between the stent (7) and adhesive (10) on the non-compliant lamellar sections A (5) is broken and the stent (7,7b) is expanded into the desired position.

When the stent (7b) is firmly fixed in the intended position within the vascular system the balloon (3) is being contracted as illustrated in Fig 5-1 (III), leaving the expanded stent (7b) in place. The non-compliant lamellar sections A (5) by being rigid helps the refolding of the balloon giving a scaffold to the (softer) compliant lamellar sections B (6), thus avoiding the dreaded "pan-caking", but also "dog-boning", while the expanded stent (7, 7a) remains in place and the adhesive parts (10) have lost their contact with the stent (7) (see for example figure 8). In case of a fluid lubricant having been added to the outer part of stent (7) and balloon (3) this lubricant may after breaking of the bond between stent (7b) and adhesive (10) now flow over the adhesive (10), helping to avoid any sticking to the vessel walls on the removal of the catheter. All of that finally allows safe removal of the balloon-catheter (1) from the final position of the stent and the vascular system.

### Example 4: Balloon with one layer being layered with stripes of a non-compliant material covered with an adhesive.

In this embodiment of the invention - depicted in Fig 6 - the medical device (1) according to the invention is a balloon catheter for stent delivery with the expandable contractible member (3) accordingly being a medical balloon. The balloon is having one (first) polymeric-layer (4). The polymeric layer is consisting of soft PEBAX^{®} with a low shore hardness thus causing a high compliance of the balloon. In addition on top of the polymeric layer (4) are laminated in at least 4 different positions in parallel to the longitudinal axis of the balloon highly non-compliant lamellar stripes being adhesive or being covered with an adhesive (10). The adhesive preferably is pressure sensitive and selected as to release the second medical device (stent) (7) from the expandable and contractible member (3) (the medical balloon) upon pressure being applied from within the second medical device aiming at distancing the stent (7) from the adhesive (10) of the balloon. Selected pressure sensitive adhesives, include silicone type pressure sensitive adhesives, acrylic type pressure sensitive adhesives and urethane type pressure sensitive adhesives. Examples of acrylic type pressure sensitive adhesives include NeoTac A-580, NeoTac A-574, NeoTac 2010, NeoTac 2457, NeoTac 2465, NeoTac 5468 all from Zeneca Resins. An example of an urethane type pressure sensitive adhesive is NeoTac 560 (Zeneca Resins). Desirably the pressure sensitive adhesive will have good water resistance to ensure good adhesion when the stent and the balloon are in contact with body fluids. The stripes may also be of metal with an adhesive on top.

Following that, the balloon is blown in a blow molding form. The balloon is then removed and crimped and folded on a catheter, thereby especially exposing the adhesive surface (10). Afterwards a metal stent (7) in non-extended state (7a) is then crimped onto the folded balloon (3), thereby allowing the adhesive surface (10) to adhere to the inner surface of the metal stent (7,/7a). If considered necessary the balloon catheter may then be treated with a lubricious material such as silicones or hydrogel polymers as well as PEO (Polyethylene oxide), NPG (neopentyl glycol diacrylate).

When introducing the balloon catheter into the vascular system the lubricious coating - if any - is facilitating movement in the system, while the stent remains fixed on the balloon stopping it from slipping-off during advancement (e.g. over a guide wire) due to the adhesive surface (10). So in Fig. 6 (I) shows - in abstract form - the situation during introduction of the medical device (1) (the balloon catheter) into a body lumen like a blood vessel. An expandable medical device (7) (a stent) in unexpanded state (7a) is disposed about the medical balloon (3). While being moved, the adhesive (10) - either fixed on top of a non-compliant stripe or being itself non-compliant - is fixing the stent (7) to the medical balloon (3), thus avoiding any slipping of the stent (7) from the balloon (3).

As illustrated in Fig. 6(II), when reaching the intended position in the vascular system the stent (7) is expanded (7b) by inflating the balloon (3). Thereby, the polymeric layer (4) does expanding more and beyond the adhesive, non-compliant stripes (10). By this expansion of the compliant layer (4) beyond the radius reached by the non-compliant adhesive stripes (10) the adhesive connection between the stent (7) and adhesive (10) is broken and the stent (7,7b) is expanded into the desired position.

When the stent (7b) is firmly fixed in the intended position within the vascular system the balloon (3) is being contracted, leaving the expanded stent (7b) in place. The non-compliant adhesive stripes (10) are rigid and thus help the refolding of the balloon giving a scaffold to the (softer) compliant balloon layer (4), thus avoiding the dreaded "pan-caking", but also "dog-boning", while the expanded stent (7, 7a) remains in place and the adhesive parts (10) have lost their contact with the stent (7). In case of a fluid lubricant having been added to the outer part of stent (7) and balloon (3) this lubricant may after breaking of the bond between stent (7b) and adhesive (10) now flow over the adhesive (10), helping to avoid any sticking to the vessel walls on the removal of the catheter (1). All of that finally allows save removal of the balloon-catheter (1) from the final position of the stent and the vascular system.

## Claims

1. A medical device (1) comprising an expandable and contractible member (3), wherein the expandable and contractible member (3) is comprising at least one first polymeric-layer (4) consisting of at least one lamellar section A (5) and an equal number of lamellar sections B (6) disposed in an alternating sequence in parallel to the longitudinal axis of the expandable and contractible member (3) with adjacent sections having a different elasticity, expressed as different shore hardness, E-module, tensile strength, elongation at break, or compliance, preferably expressed as a different shore hardness.

2. A medical device (1) according to claim 1, comprising an expandable and contractible member (3), wherein the expandable and contractible member (3) is comprising at least one first polymeric-layer (4) consisting of at least one lamellar section A (5) and an equal number of lamellar sections B (6) disposed in an alternating sequence in parallel to the longitudinal axis of the expandable and contractible member (3) with adjacent sections having a different shore hardness, E-module, tensile strength, elongation at break, or compliance, preferably a different shore hardness.

3. The medical device according to any of claims 1 or 2, wherein the expandable and contractible member (3) is a medical balloon.

4. The medical device according to any of claims 1 to 3, wherein the first polymeric-layer (4) of the first expandable and contractible member (3) is consisting of at least 2 each of lamellar sections A (5) and lamellar sections B (6), preferably of at least 3 each of lamellar sections A (5) and lamellar sections B (6).

5. The medical device according to any of claims 1 to 4, wherein lamellar section A (5) and lamellar section B (6) are consisting of two different polymeric materials.

6. The medical device according to any of claims 1 to 5, wherein the medical device (1) is a delivery apparatus for delivering at least one second medical device (7).

7. The medical device according to claim 6 comprising as second medical device (7) an expandable medical device disposed about the first expandable and contractible member (3).

8. The medical device according to claim 7, wherein the expandable medical device (7) is a stent, a stent graft, a graft or a graft connector, preferably is a stent .

9. The medical device according to any of claims 1 to 8, wherein the expandable and contractible member (3) is comprising an additional second polymeric-layer (8) disposed within the first polymeric layer (4).

10. The medical device according to any of claims 1 to 8, wherein the expandable and contractible member (3) is comprising an additional third polymeric-layer (9) disposed about the first polymeric layer (4).

11. The medical device according to any of claims 9 or 10, wherein the expandable and contractible member (3) is comprising an additional third polymeric-layer (9) disposed about the first polymeric layer (4) and an additional second polymeric-layer (8) disposed within the first polymeric layer (4).

12. The medical device according to any of claims 9 or 11, wherein the second polymeric-layer (8) is consisting of PEBA or Nylon or mixtures thereof.

13. The medical device according to any of claim 5, wherein either the polymeric material, of which lamellar section A (5) or lamellar section B (6) consists, is adhesive, while the other is not, or one of lamellar section A (5) or lamellar section B (6) is layered with an adhesive (10), while the other is not.

14. The medical device according to any of claim 1 to 13, wherein the polymeric material, of which one of lamellar sections A (5) or B (6) consists, is highly non-compliant while the other polymeric material, of which the other of lamellar sections A (5) or B (6) consists, is highly compliant.

15. A method for improving the refolding behaviour of a first expandable and contractible member (3) of a medical device (1) comprising providing at least one first polymeric-layer (4) consisting of at least one lamellar section A (5) and an equal number of lamellar sections B (6) disposed in an alternating sequence in parallel to the longitudinal axis of the expandable and contractible member (3) with adjacent sections having a different elasticity.

16. The method according to claim 15, wherein the adjacent sections are having a different elasticity, expressed as different shore hardness, E-module, tensile strength, elongation at break, or compliance, preferably expressed as a different shore hardness.

17. The method according to any of claims 15 or 16, wherein the first expandable and contractible member (3) is a medical balloon.

18. The method according to any of claims 15 to 17, wherein the first polymeric-layer (4) of the first expandable and contractible member (3) is consisting of at least 2 each of lamellar sections A (5) and lamellar sections B (6), preferably of at least 3 each of lamellar sections A (5) and lamellar sections B (6).

19. The method according to any of claims 15 to 18, wherein lamellar section A (5) and lamellar section B (6) are consisting of two different polymeric materials.

20. The method according to any of claims 15 to 19, comprising additionally providing an additional second polymeric-layer (8) of the expandable and contractible member (3) disposed within the first polymeric layer (4).

21. The method according to any of claims 15 to 19, comprising additionally providing an additional third polymeric-layer (9) of the expandable and contractible member (3) disposed about the first polymeric layer (4).

22. The method according to any of claims 15 to 19, comprising additionally
• providing an additional third polymeric-layer (9) of the expandable and contractible member (3) disposed about the first polymeric layer (4) and
• providing an additional second polymeric-layer (8) of the expandable and contractible member (3) disposed within the first polymeric layer (4).

23. The method according to any of claims 20 or 22, wherein the second polymeric-layer (8) is consisting of PEBA or Nylon or mixtures thereof.

24. The method according to claim 19, wherein either the polymeric material, of which lamellar section A (5) or lamellar section B (6) consists, is adhesive, while the other is not, or one of lamellar section A (5) or lamellar section B (6) is layered with an adhesive, while the other is not.

25. The method according to any of claims 19, wherein the polymeric material, of which one of lamellar sections A (5) or B (6) consists, is non-compliant while the other polymeric material, of which the other of lamellar sections A (5) or B (6) consists, is compliant.

26. A method of producing a medical device according to any of claims 1 to 14, wherein the lamellar section/s A (5) and the lamellar section/s B (6) are co-extruded, when producing the first polymeric-layer (4) of the expandable and contractible member (3).

27. A method of producing a medical device according to any of claims 9 or 11, wherein the lamellar section/s A (5) and the lamellar section/s B (6) of the expandable and contractible member (3) are either simultaneously or consecutively extruded onto the second polymeric-layer (8).
